# EUROPEAN PATENT SPECIFICATION

(11) **EP 4 096 747 B1**
(45) Date of publication and mention of the grant of the patent: **23.07.2025**
(21) Application number: 20830380.0
(22) Date of filing: 23.12.2020
(51) Int. Cl.: A61M 5/14, A61M 5/142, A61M 5/145

(54) **PUMP FOR DELIVERING LIQUID TO BE ADMINISTERED INTRAVENOUSLY**
PUMPE ZUR ABGABE EINER INTRAVENÖS ZU VERABREICHENDEN FLÜSSIGKEIT
POMPE DE DISTRIBUTION DE LIQUIDE À ADMINISTRER PAR VOIE INTRAVEINEUSE

(30) Priority: 27.01.2020 EP 20153930
(43) Date of publication of application: 07.12.2022
(73) Proprietor: Ivy Medical Holding B.V., 9743 AJ Groningen (NL)
(72) Inventor: TUIN, Peter Rieko, 9743 AJ Groningen (NL)
(74) Representative: V.O.
(86) International application number: PCT/NL2020/050816
(87) International publication number: WO 2021/154068

(56) References cited:
- WO-A1-2016/089776
- US-A- 4 396 385
- US-A- 4 850 980
- US-A- 5 658 133

## Description

### FIELD AND BACKGROUND OF THE INVENTION

The invention relates to a pump for delivering an intravenous ("IV") solution or other liquid, such as a medicament, to be administered intravenously.

Intravenous administration of a liquid, usually a solution, is commonly carried out by delivery from an IV bag using pressure resulting from gravity. To create and maintain pressure resulting from gravity, the IV bag is suspended at a level higher than the level range of the patient body, for instance from a pole projecting from a foot or mounted to a bed. Such a pole severely restricts the freedom of movement of the patient. IV administration of medication may be required for prolonged periods of time. Restricting mobility of a patient for a prolonged period of time is inconvenient for the patient, increases the need of nursery care and is particularly disadvantageous since it has been found that physical exercise and mobility has a positive effect on patient health, so that restricting patient mobility has an adverse effect on patient health and tends to lead to longer stays in hospital.

Delivery of an IV solution may be supported by a peristaltic or piston driven IV pump. Use of such pumps is generally indicated if a specific amount of a pharmacologic agent is to be administered and/or fluid overload is to be prevented. However, such pumps are not suitable to be worn on the patient body due to weight, size and certified use restrictions and usually mounted to an IV pole, so that no improvement regarding patient mobility is achieved. Furthermore, such IV pumps are costly.

US 4,396,385 A discloses that a metering apparatus for controlling the flow of fluid through a fluid infusion system at an operator-designed rate includes a single-use cassette having a syringe-type pump in the flow system.

US 4,850,980 A discloses that a cassette for use with an IV infusion pump has a housing with a first inlet, a second inlet, an outlet, and a port to the IV infusion pump.

WO 2016/089776 A1 discloses apparatus, systems and methods of delivering medical fluid to patients. More particularly, infusion pumps, disposable cassettes and associated methods are disclosed.

### SUMMARY OF THE INVENTION

It is an object of the invention to provide a pump for providing liquid to be delivered intravenously that is wearable and can be used safely, easily and economically in a health care environment. More in particular, it is an object to provide a pump that is compatible with conventional IV bags and which accurately delivers IV liquid at a predetermined flow rate, regardless the orientation of the pump.

According to the invention, this object is achieved by providing a device according to claim 1. The invention can also be embodied in a disposable unit according to claim 13 for use in a base unit of such a pump.

In use of such a device, the parts of the pump that are in direct contact with infusion liquid, in particular the pump cylinders and the pump valve, and which need to be sterile at least when perfusion is started, are replaceable as a single disposable unit, which is replaceable very easily. The base unit of the pump, in particular the relatively costly pump drive and the valve actuators can be re-used many times, so that costs are saved and relatively little waste is produced.

Particular elaborations and embodiments of the invention are set forth in the dependent claims. Features of the dependent claims applying to the disposable unit can also be applied advantageously in a disposable unit according to claim 13.

Further features, effects and details of the invention appear from the detailed description and the drawings.

### BRIEF DESCRIPTION OF THE DRAWINGS

Fig. 1 is a perspective view of a first example of an IV pump according to the invention;
Fig. 2 is a perspective view of the IV pump shown in Fig. 1 with a lid opened;
Fig. 3 is a perspective exploded view of the IV pump as shown in Fig. 2 without an exchangeable and disposable cylinder and valve arrangement;
Fig. 4 is a cross-sectional view along a plane IV-IV in Fig. 5 of the exchangeable and disposable cylinder and valve arrangement;
Fig. 5 is a cross-sectional view along a plane V-V in Fig. 4 of the exchangeable and disposable cylinder and valve arrangement in a first operating condition;
Fig. 6 is a cross-sectional view along a plane V-V in Fig. 4 of the exchangeable and disposable cylinder and valve arrangement in a second operating condition;
Fig. 7 is a perspective view of a second example of an IV pump according to the invention;
Fig. 8 is a perspective view of the IV pump shown in Fig. 7 with a lid opened;
Fig. 9 is a top-plan view of the pump shown in Figs. 7 and 8 with an exchangeable and disposable cylinder and valve arrangement in a first operating condition;
Fig. 10 is a top-plan view of the pump shown in Figs. 7-9 with the exchangeable and disposable cylinder and valve arrangement in a second operating condition; and
Fig. 11 is a perspective view of the IV pump as shown in Fig. 8 without the exchangeable and disposable cylinder and valve arrangement.

### DETAILED DESCRIPTION

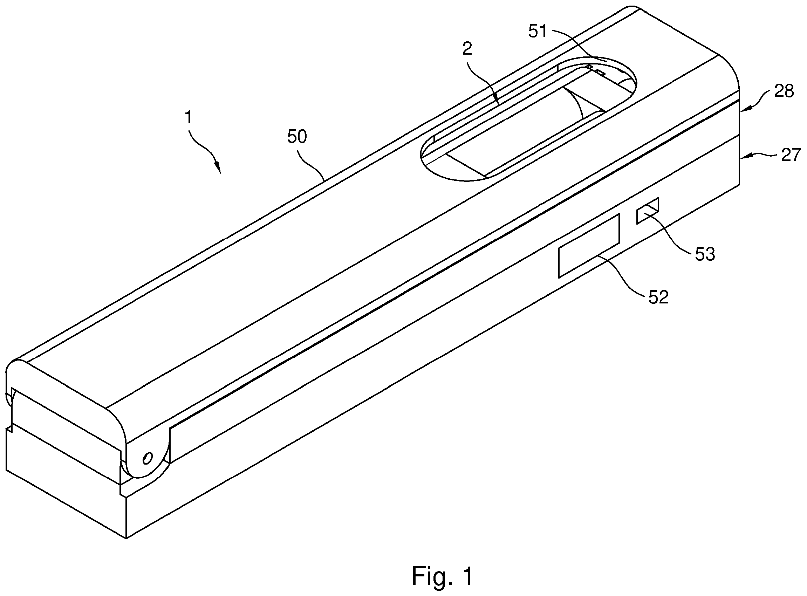

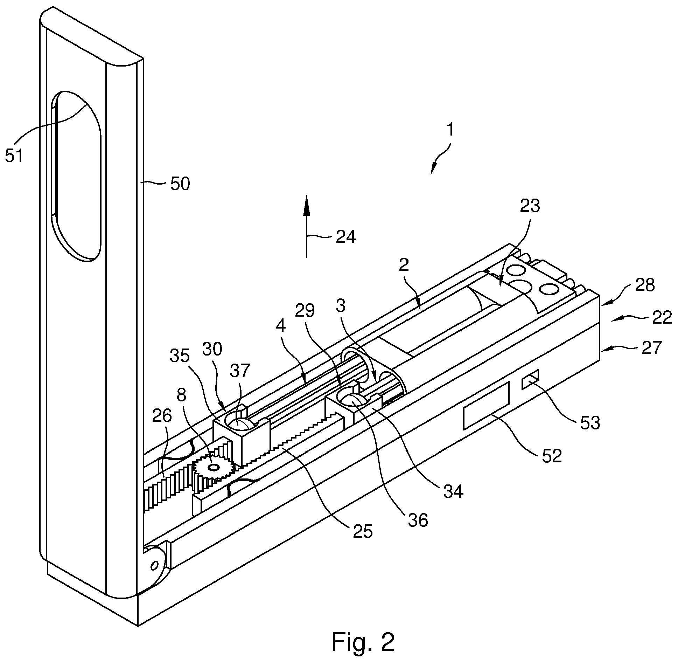

In Figs. 1-6, a first example of an infusion delivery pump 1 according to the invention is shown. The pump 1 has a pump housing 2 and two plungers 3, 4. The pump housing 2 and the plungers 3, 4 bound pump chamber 5 and, respectively 6. The plungers 3, 4 are reciprocally movable inwardly and outwardly of the pump housing 2 thereby increasing and decreasing the volume of the pump chambers 5 and, respectively, 6.

For driving displacement of the plungers relative 3, 4 to the pump housing 2, a plunger drive 7 is provided. The plunger drive 7 has a pinion wheel 8 in driving engagement with toothed racks 25, 26 which are coupled to respective ones of the plungers 3, 4. The plunger drive 7 is further composed of a shaft 9 engaging a coupling member 10 of an electric motor 11.

The pump 1 further has two supply conduit sections 12, 13 merging into a common supply conduit section 14 which communicates with one of the pump chambers 5, 6 for delivering infusion liquid into that pump chamber 5 or 6.

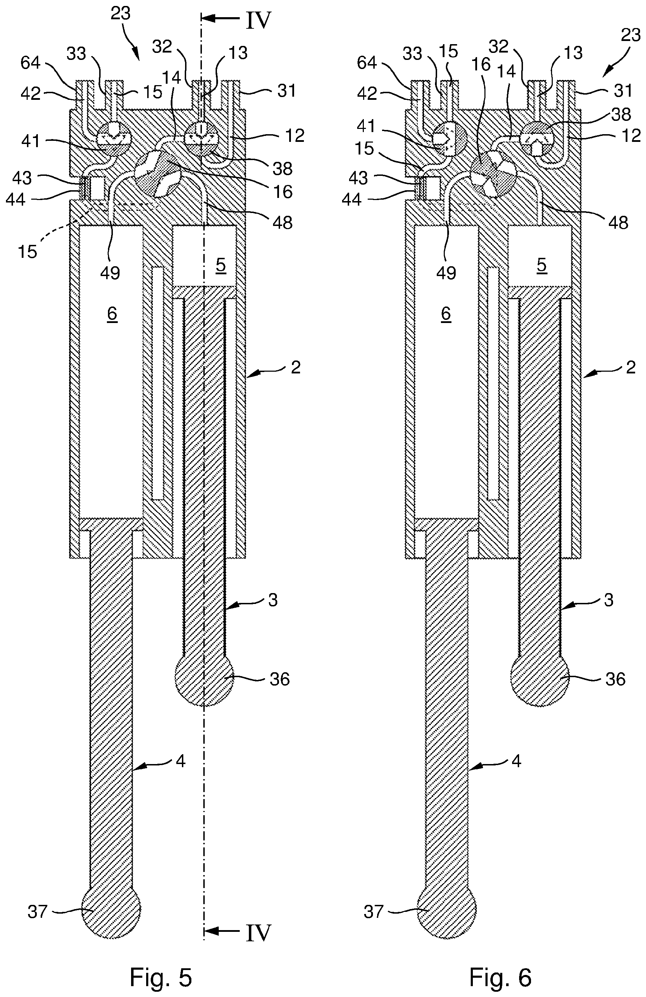

In the operating condition shown in Fig. 5, a delivery conduit 15 communicates with the other one of the pump chambers 5 or 6 for receiving and delivering infusion liquid from that pump chamber 5, 6.

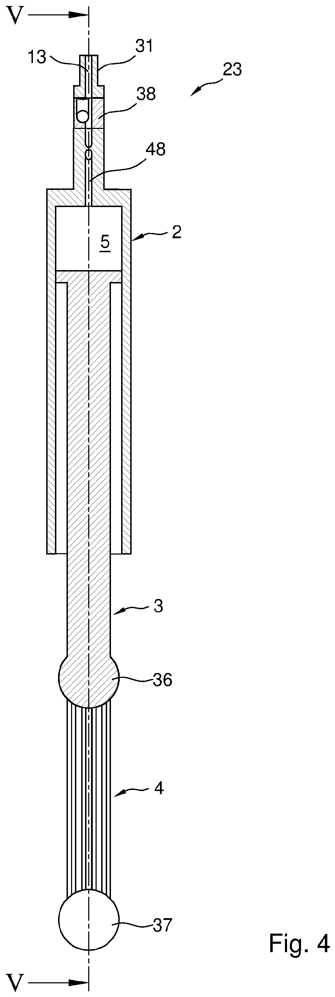

The supply conduit sections 12 or 13 and 14 communicate with one of the pump chambers via pump valve 16, while the delivery conduit 15 is shut off from that one of the pump chambers 5, if the pump valve 16 is in an infusion intake condition with regard to that pump chamber 5 as is shown in Figs. 4 and 5. If the pump valve 16 is in an infusion delivery condition with regard to the pump chamber 5, as is shown in Fig. 6, the delivery conduit 15 communicates with the pump chamber 6, while the supply conduit sections 12-14 are shut off from that pump chamber 6. For operating the pump valve 16, a pump valve actuator 17 is provided. The pump valve actuator 17 has a pump valve coupling member 18 for coupling the pump valve actuator 17 to the pump valve 16, an electric pump valve motor 20 and a pump valve motor coupling member 19 coupling the pump valve coupling member 18 to the pump valve motor 20.

A control unit 21 is arranged and connected for controlling the motor 11 of the plunger drive 7 and motor 20 of the pump valve actuator 17.

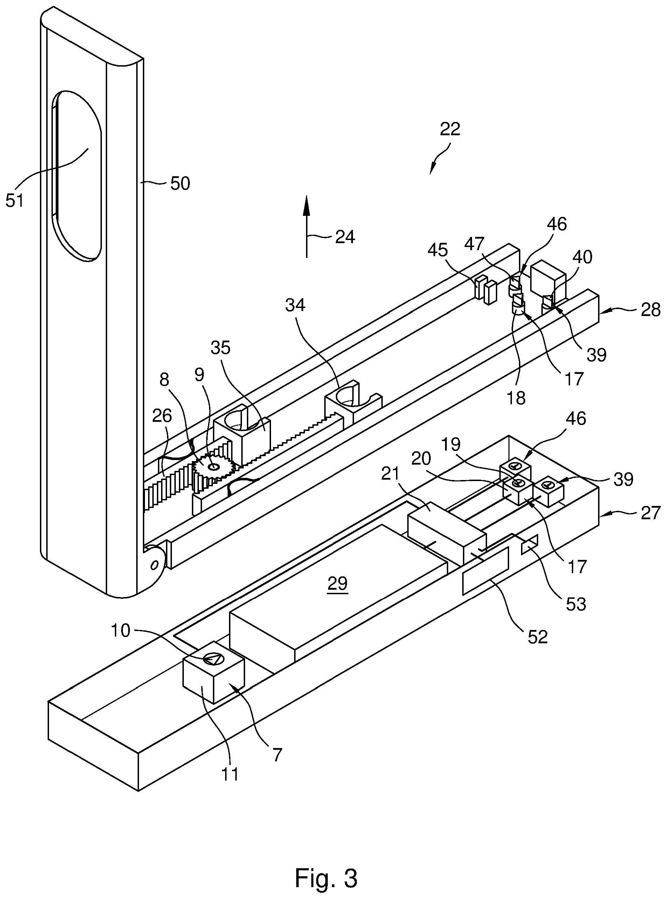

The plunger drive 7, the pump valve actuator 17 and the control unit 21 are part of a base unit 22 (shown separately in Fig. 3), while the pump housing 2, the plungers 3, 4, the supply conduit sections 12-15 and the delivery conduit 15 and the pump valve 16 are part of a disposable unit 23 (shown separately in Figs. 4-6) which is removably attached to the base unit 22. The coupling of the pump valve 16 to the pump valve actuator 17 and the coupling of the plungers 3, 4 to the plunger drive 7 are releasable.

This allows the parts of the pump 1 that are in direct contact with infusion liquid, in particular the pump cylinders and the pump valve, and which need to be sterile at least when perfusion is started, to be replaceable as a single disposable unit, which can be mounted and dismounted very easily. The rest of the pump 1, in particular the relatively costly plunger drive 7 and the valve actuators 17, 39, 40 can be re-used many times, so that costs are saved and relatively little waste is produced.

The base unit 22 has a lid 50 pivotably mounted to a main portion of the base unit 22. The lid 50 shields the mounted disposable unit 23, while a window 51 in the lid 50 allows visual inspection of the position of the plungers 3, 4 and, if the pump housing 2 is transparent at least between the window 51 and the chambers 5, 6, visual inspection of the liquid in the pump chambers 5, 6 in the housing 2.

In the present example, the base unit 22 has a bottom portion 27 and a top portion 28. In normal use, the bottom portion 27 remains attached to the top portion 28, so that the coupling member 10 remains connected to the shaft 9 and the pump valve motor coupling member 19 remains coupled to the pump valve coupling member 18. Only for repairs, such as exchanging a rechargeable battery 29, the bottom portion 27 and the top portion 28 of the base unit 22 are dismounted from each other.

For showing an operating condition of the pump 1, a display 52 connected to the control unit 21 is provided. For charging the battery 29 and for connecting the control unit 21 to an external device, a port 53 is provided, which may for instance be an USB-C port. This allows to display and control further features of operation and settings of the pump 1 via the external device. Data regarding operation and settings of the pump may also be monitored and/or registered by the external device, for instance to verify flow rates of liquid that have been administered to a patient during a given period of use. A transmitter/receiver for wireless communication between the control unit 21 and an external device may also be provided.

Removing a used disposable unit 23 from the base unit 22 and mounting a fresh disposable unit to the base unit 22 is made particularly easy, because the coupling of the pump valve 16 to the pump valve actuator 17 and the coupling of the plungers 3, 4 to the plunger drive 7 are releasable in one common direction 24. In the present example, this is achieved by providing couplings of the plungers 3, 4 to the toothed racks 25, 26 of the plunger drive 7 in the form of releasable ball joint connections 29, 30 of which outer members 34, 35 are open in the releasing direction 24 transverse to the direction in which plungers 3, 4 are movable, so that ball members 36, 37 can be moved out of engagement with the outer members 34, 35. Preferably, the opening of the outer members 34, 35 in the releasing direction 24 is slightly smaller than the contour of the ball members 36, 37, so that a small resistance has to be overcome to mount and dismount the ball joint connections 29, 30. Other releasable connections, such as T-connections or pin-hole connections, are also conceivable.

An advantage of ball joint connections 29, 30 is that during mounting, the disposable unit 23 can be oriented in a wide range of directions relative to the base unit 22 while the disposable unit 23 is connected to the base unit 22 only via one or both of the ball joint connections 29, 30. This facilitates mounting and dismounting of the disposable unit 23 and avoids damage to the coupling of the plungers 3, 4 to the plunger drive 7 if the disposable unit 23 is rotated relative to the base unit 22 while not fully disconnected at the coupling of the plungers 3, 4 to the plunger drive 7.

The supply conduit sections 12, 13 and the delivery conduit 15 have end portions 31-33 distally from the pump chambers 5, 6 that are arranged for connection to infusion transfer tubing. In this example, the end portions are provided in the form of projections 31-33 insertable into ends of the tubing for frictional engagement with the tubing ends that are slightly expanded by the ends 31-33 inserted therein.

The two upstream supply conduit sections 12, 13 in the disposable unit 23 are each provided with a connection 31, 32 for infusion transfer tubing and merge into a downstream section 14. A three-way source selector valve 38 is provided between the upstream supply conduit sections 12, 13 and the downstream supply conduit section 14 for selectively causing a first or a second one of the upstream supply conduit sections 12, 13 to communicate with the downstream supply conduit section 14. The base unit 22 is provided with a source selector valve actuator 39 with a coupling 40 releasably coupled to the source selector valve 38. Operating the source selector valve 38 allows switching between supply from a first source communicating with the supply conduit section 12 (see Fig. 6) and supply from a second source communicating with the supply conduit section 13 (see Fig. 5). The first source may for instance be an IV bag filled with standard IV solution while the second source may for instance be a container holding a liquid medicament. During filling of the pump chamber 5 or 6 by means of a given displacement of the plunger 3 or 4, supplying from the second source during a predetermined portion of that displacement of the plunger 3 or 4 allows to mix in a predetermined dosage volume or concentration of the medicament to be administered into the total volume of liquid supplied into the pump chamber. In the pump chamber 5 or 6, the medicament is then, at least to a certain extent, mixed with the IV solution and can subsequently be administered to the patient during delivery of the supplied liquid from the pump chamber 5 or 6 into which it has been supplied.

The source selector valve actuator 39 is also connected to the control unit 21 which is arranged for causing the source selector valve actuator 39 to be actuated in accordance with a dosage volume or mixing ratio setting and displacement of the plungers 3, 4 during filling of the respective pump chambers 5, 6.

Because the source selector valve 38 is also included in the disposable unit 23 and releasably coupled to the source selector valve actuator 39 of the base unit 22, also the coupling of the source selector valve 38 and the source selector valve actuator 39 is made and released as the disposable unit 23 is mounted or dismounted.

In the present example, the source selector valve 38 is a three-way valve that can be switched between a first operating position providing open communication between the first supply conduit section 12 and the common supply conduit section 14 while blocking supply from the second supply conduit section 13 to the common supply conduit section 14 and a second operating position providing open communication between the second supply conduit section 13 and the common supply conduit section 14 while blocking supply from the first supply conduit section 12 to the common supply conduit section 14. This allows controlling dosage volumes or concentration using a single valve. It is however also possible to provide separate valves for opening and closing the first and second supply conduit sections. Furthermore, it is also possible to provide three or more upstream supply conduit sections which associated tubing connections to allow mixing from three or more sources.

The disposable unit 23 is further provided with a three-way bleeding valve 41 in the delivery conduit 15, a bleeding conduit 42 branching off from the delivery conduit 15 at the bleeding valve 41, a bubble detector 43 and a bubble detector signal output port in the form of contact 44. The base unit 22 is further provided with a bubble detector signal input port in the form of contact 45 in communication with the bubble detector signal output port 44 and a bleeding valve actuator 46 with a coupling 47 releasably coupled to the bleeding valve 41. The control unit 21 is connected and arranged for operating bleeding valve actuator 46 for causing the bleeding valve 41 to switch, in response to a signal from the bubble detector 43 representing detection of presence of air in liquid being delivered from the pump chamber 5 or 6 through the delivery conduit 15, from a position in which the delivery conduit 15 is open until its downstream end to a position in which the bleeding conduit 42 communicates via the bleeding valve 41 with the portion of the delivery conduit 15 upstream of the bleeding valve 41 and the one of the pump chambers 5, 6 from which liquid is delivered. Thus, detected air is discharged via the bleeding conduit 42 together with a small amount of the liquid. If no further air is detected, first the piston 3 or 4 is displaced over a small further distance, to allow for displacement of the detected air from the bubble detector 43 until downstream of the bleeding valve 41, and then the bleeding valve 41 is switched back to the position shown in Fig. 6 for resuming delivery of liquid via the delivery conduit 15. Thus, bubbles can reliably be evacuated from the liquid to be administered.

In this example the bleeding conduit opens into the environment at a connection 64. However, a tube for discharging liquid spilled during bleeding or a receptacle or an absorbing body for receiving such spilled liquid may be provided to avoid spilling of liquid outside of the pump.

During priming of the pump chamber 5, the plunger 3 can first be moved outwardly with the pump valve in the position shown in Fig. 6, in which the pump chamber 5 communicates with one of the supply conduit sections 12 and 13 and with the common supply conduit section 14, so that the internal volume of the first pump chamber 5 is increased and liquid is supplied into the first pump chamber 5. Then, the plunger 3 is stopped and the pump valve 16 is switched into the position shown in Fig. 5, in which the pump chamber 5 communicates with the delivery conduit 15. With the bleeding valve 41 in the position shown in Fig. 5, in which the pump chamber 5 communicates with the bleeding conduit 42 and while holding the pump 1 in an orientation in which the transfer conduit 48 opens into an upper end of the pump chamber 5, the plunger 3 is subsequently moved inwardly, so that the internal volume of the pump chamber 5 is reduced and air and priming liquid are expelled through the bleeding conduit 42. The other pump chamber 6 can be primed in a corresponding manner.

Instead of by a bubble detection and diversion system, bubble evacuation may also be carried out passively, for instance as in a conventional drip chamber.

The valves 16, 38, 41 are rotary valves each having a plug rotatable in the pump housing 2, the plugs each have passages arranged for providing communication between an inlet one and an outlet one of ports in the pump housing that are facing the plug in selected rotary positions of the plug. The plugs have axes of rotation in the direction 24 in which the disposable part 23 is releasable from the base part 22. This allows the valves 16, 38 and 41 in the disposable unit 23 to be operated by and to be releasably coupled to the actuators 17, 39 and 46 in the base unit 22 in a simple manner. In the present example, all valves are three-way or four-way versions of such rotary valves. It is however also possible to provide the disposable unit with only one or any other number of such valves and to provide one or more of these valves with a different number of ports, e.g. just two ports, for instance in the form of a stopcock valve, or a larger number of ports, for instance for bleeding directly from the pump valve.

To ensure that the position of the valve actuators 17, 39 and 46 are associated to positions of the plugs of the valves 16, 38 and, respectively, 41 in a one on one relationship only, the plugs and the valve actuators 17, 39 and 46 coupled thereto have complementary shaped coupling faces, which are each non-rotation symmetrical about the axis of rotation of the respective plug. Thus, for each valve plug, there is only one relative rotational position in which the associated valve actuator coupling 18, 40 and 47 can be coupled thereto. In the present example, the valve actuator couplings 18, 40, 47 each have a triangular projection of which the center is offset from the axis of rotation of the respective valve actuator coupling 18, 40, 47 and the plugs have matching triangular recesses in which the triangular projections only fit closely if the orientations of the valve actuator couplings 18, 40, 47 match the orientations of the plugs. The triangular projections visually resemble arrows, which facilitates checking whether the orientations of the valve actuator couplings 18, 40, 47 match the orientations of the plugs before mounting a disposable unit 23 to a base unit 22.

Because the pump 1 according to the present example has two of the plungers 3, 4 and the pump housing 2 and the plungers 3, 4 bounds two pump chambers 5, 6 and both plungers 3, 4 are reciprocally movable inwardly and outwardly of the pump housing 2, thereby increasing and decreasing the volume of the respective pump chamber 5, 6 and the plunger drive 7 is arranged for simultaneously driving the plungers 3, 4 in opposite directions, a single drive is sufficient for driving two plungers for supplying IV liquid to one of the pump chambers 5, 6 and delivering IV liquid from the other one of the pump chambers 5, 6.

In the pump 1 according to the present example, the plungers 3, 4 are coupled to each other for simultaneous movement in mutually opposite directions only. Thus, supplying IV liquid to one of the pump chambers 5, 6 and simultaneously delivering IV liquid from the other one of the pump chambers 5, 6 is achieved in a simple manner.

Because the pump valve 16 is four way valve in which ports are arranged for alternatingly causing one of the pump chambers 5, 6 to communicate with the delivery conduit and causing the other one of the pump chambers 5, 6 to communicate with the supply conduit and vice versa, operation of a single valve is sufficient for switching between supplying liquid to a first one of the pump chambers 5, 6 while delivering liquid from a second one of the pump chambers 5, 6 to supplying liquid to the second one of the pump chambers 5, 6 while delivering liquid from the first one of the pump chambers 5, 6.

The pump valve 16 is arranged so that there is no direct communication between, on the one hand, the supply conduit sections 12 or 13 and 14 and, on the other hand, the delivery conduit 15 in any position of the pump valve 16. Thus, uncontrolled supply of IV liquid bypassing the pump chambers 5, 6 is avoided, even if for instance the disposable unit 22 is dismounted without disconnecting the patient from the pump 1 or pressure is exerted onto the IV bag (e.g. as a result of falling). This is particularly useful for avoiding inadvertent direct and undiluted administration of a medicament that has been mixed in.

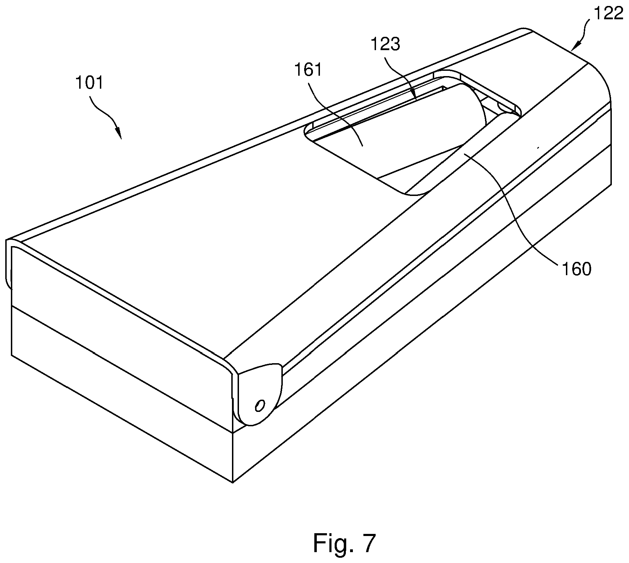

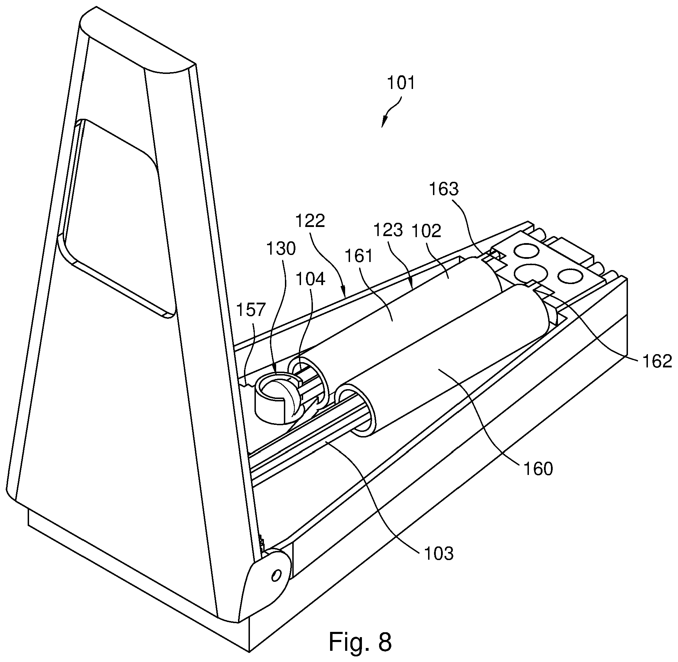

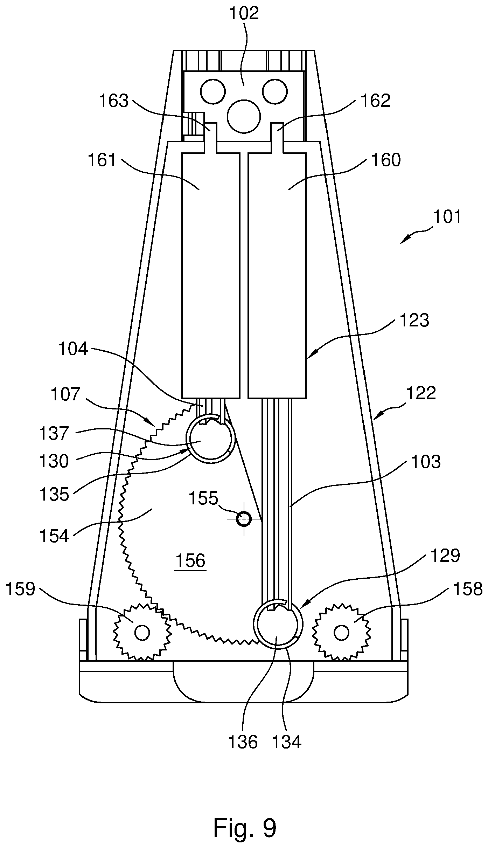

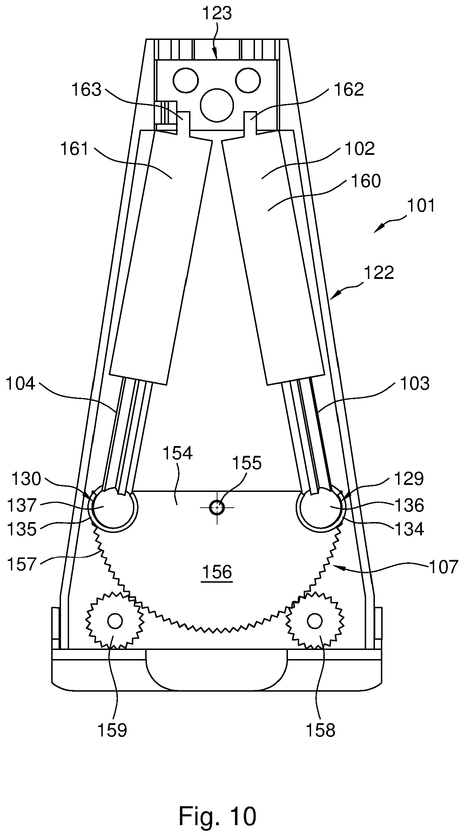

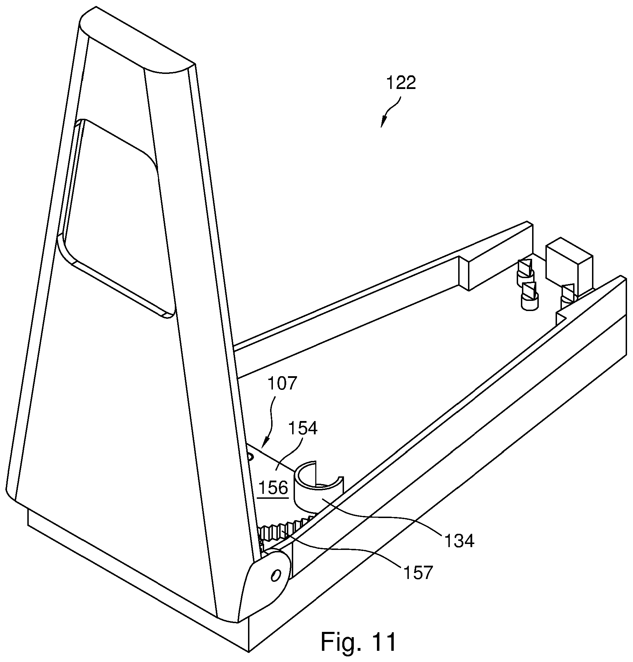

In Figs. 7-11, a second example of a pump 101 according to the invention is shown. Also this pump 101 is composed of a re-usable base unit 122 and a disposable unit 123. In Fig. 11, the re-usable base unit 122 is shown without the disposable unit 123 mounted thereto.

The plungers 103, 104 are coupled to a double arm 154 having an axis of rotation 155 extending centrally between the plungers 103, 104. Thus, the coupling of the plungers 103, 104 to each other is achieved in a very simple manner and the plungers 103, 104 are guided by the double arm 154 with very little friction.

The double arm 154 is a portion of a gear wheel 156 having a toothed gear rack 157 extending along at least a section of a circle concentric with the axis of rotation 155 of the double arm 154. The plunger drive 107 further has pinions 158, 159 coupled to an electric motor. The pinions 158, 159 drivingly engaging the toothed gear rack 157. Thus, pivoting of the double arm 154 about the axis of rotation, and thereby simultaneous movement of the plungers 103, 104 in opposite directions is driven in a simple manner. A particular advantage of driving movement of the plungers via a double arm is that the pump 101 can be relatively short in longitudinal direction, because of the absence of a toothed rack projecting from the plungers in the direction of movement of the plungers.

To allow the orientations of the pump chambers to accommodate to the movement of ends of the plungers 103, 104 along a circle segment, the pump housing 102 includes two pump housing parts 160, 161 each bounding one of the pump chambers, that are pivotably suspended near ends 162, 163 distally from the couplings 129, 130 engaging the plungers 103, 104.

Also in this example, the couplings 129, 130 of the plunger drive 107 to the plungers 103, 104 are provided in the form of ball joints of which the balls 136, 137 are releasable from outer coupling members 134, 135. In this example, a further advantage of the ball joints 129, 130 is that angular movement at the couplings 129, 130 during pivoting of the double arm 154 is also accommodated.

Several features have been described as part of the same or separate embodiments. However, it will be appreciated that the scope of the invention also includes embodiments having combinations of all or some of these features other than the specific combinations of features embodied in the examples.

## Claims

1. An infusion delivery pump (1) comprising:
a pump housing (2);
a plunger (3), wherein the pump housing and the plunger bound a pump chamber (5), the plunger is reciprocally movable inwardly and outwardly of the pump housing thereby increasing and decreasing the volume of the pump chamber;
a plunger drive (7) for driving displacement of the plunger relative to the pump housing, the plunger drive being coupled to the plunger;
a supply conduit (12-14) communicating with the pump chamber for delivering infusion liquid from a reservoir, such as an IV bag, into the pump chamber;
a delivery conduit (15) communicating with the pump chamber for receiving and delivering infusion liquid from the pump chamber;
at least one pump valve (16) via which the supply conduit communicates with the pump chamber, while the delivery conduit is shut off from the pump chamber, if the pump valve is in an infusion intake condition and via which the delivery conduit communicates with the pump chamber, while the supply conduit is shut off from the pump chamber, if the pump valve is in an infusion delivery condition;
at least one pump valve actuator (17); and
a control unit (21) arranged and connected for controlling the plunger drive (7) and the pump valve actuator (17);
wherein the plunger drive (7), the pump valve actuator (17) and the control unit (21) are part of a base unit (22);
wherein the pump housing (2), the plunger (3), at least a downstream portion of the supply conduit and at least one upstream portion of the delivery conduit and the at least one pump valve are part of a disposable unit removably attached to the base unit, the coupling of the at least one pump valve to the at least one pump valve actuator and the coupling of the at least one plunger to the plunger drive being releasable,
**characterized by**:
a further plunger (4), wherein the pump housing (2) and the further plunger bound a further pump chamber (6), the further plunger is reciprocally movable inwardly and outwardly of the pump housing thereby increasing and decreasing the volume of the further pump chamber, and wherein the plunger drive (7) is arranged for simultaneously driving the plungers (3, 4) in opposite directions.

2. A pump (1) according to claim 1, wherein the coupling of the at least one pump valve (16) to the at least one pump valve actuator (17) and the coupling of the at least one plunger (3, 4) to the plunger drive (7) are releasable in one common direction.

3. A pump (1) according to claim 1 or 2, wherein the supply conduit (12-14) and the delivery conduit (15) have ends distally from the pump chamber (5) that are arranged for connection of infusion transfer tubing.

4. A pump (1) according to any one of the preceding claims, wherein, in the disposable unit (23), the supply conduit (12-14) comprises at least two upstream supply conduit sections, each with a connection for infusion transfer tubing, and one downstream common supply conduit section, a source selector valve (38) being provided for selectively causing a first or a second one of the upstream supply conduit sections to communicate with the common supply conduit section, and wherein the base unit (22) further comprises a source selector valve actuator comprising a coupling releasably coupled to the source selector valve.

5. A pump (1) according to claim any of the preceding claims, wherein the disposable unit (23) further comprises a bleeding valve in the delivery conduit, a bleeding conduit branching off from the delivery conduit at the bleeding valve, a bubble detector and a bubble detector signal output port, and wherein the base unit further comprises a bubble detector signal input in communication with the bubble detector signal output port and a bleeding valve actuator releasably coupled to the bleeding valve, wherein the control unit is connected and arranged for operating the bleeding valve in response to a signal from the bubble detector representing detection of presence of air so that detected air is discharged via the bleeding conduit.

6. A pump (1) according to any of the preceding claims, wherein at least one of said at least one pump valve is a rotary valve with a plug rotatable in the pump housing, the plug having passages arranged for providing communication between an inlet one and an outlet one of ports facing the plug in at least one rotary position of the plug, the plug having an axis of rotation in a direction in which the disposable unit is releasable from the base unit, and wherein the plug can be operated by and be releasably coupled to a corresponding one of said at least one pump valve actuators.

7. A pump (1) according to claim 6, wherein the plug, on the one hand, and the corresponding pump valve actuator that can be releasably coupled to the plug, on the other hand, have complementary shaped coupling faces, which are non-rotation symmetrical about the axis of rotation of the plug, in such manner that there is only one relative rotational position in which the corresponding pump valve actuator can be coupled to the plug.

8. A pump (1) according to any of the preceding claims, wherein the plungers (3, 4) are coupled to each other for simultaneous opposite movement only, so that liquid is delivered from a pump chamber of one of the plungers only while liquid is supplied to a pump chamber of the other one of the plungers.

9. A pump (1) according to claim 8, wherein the plungers (3, 4) are coupled to a double arm having an axis of rotation extending centrally between the plungers.

10. A pump (1) according to claim 9, wherein the double arm is a portion of a gear wheel having a toothed gear rack extending along at least a section of a circle concentric with the axis of rotation of the double arm, the plunger drive further comprising a pinion drivingly engaging the toothed gear rack.

11. A pump (1) according to claim 9 or 10, wherein the pump housing (2) includes two pump housing parts each bounding one of the pump chambers (5, 6), wherein the pump housing parts are pivotably suspended near ends distally from the couplings engaging the plungers (3, 4).

12. A pump (1) according to any of the preceding claims, wherein the pump valve is a four way valve in which ports are arranged for alteratingly causing one of the pump chambers (5, 6) to communicate with the delivery conduit and causing the other one of the pump chambers to communicate with the supply conduit and vice versa.

13. A disposable unit (23) for use in an infusion delivery pump (1), the disposable unit comprising:
a pump housing (2);
a plunger (3), wherein the pump housing and the plunger bound a pump chamber (5), the plunger is reciprocally movable inwardly and outwardly of the pump housing thereby increasing and decreasing the volume of the pump chamber;
a supply conduit (12-14) communicating with the pump chamber for delivering infusion liquid from a reservoir, such as an IV bag, into the pump chamber;
a delivery conduit (15) communicating with the pump chamber for receiving and delivering infusion liquid from the pump chamber; and
at least one pump valve (16) via which the supply conduit communicates with the pump chamber, while the delivery conduit is shut off from the pump chamber, if the pump valve is in an infusion intake condition and via which the delivery conduit communicates with the pump chamber, while the supply conduit is shut off from the pump chamber, if the pump valve is in an infusion delivery condition;
wherein the at least one pump valve (16) is arranged for releasable coupling to at least one pump valve actuator (17) and the at least one plunger is arranged for releasable coupling to a plunger drive (7),
**characterized by**:
a further plunger (4), wherein the pump housing (2) and the further plunger (4) bound a further pump chamber (6), the further plunger is reciprocally movable inwardly and outwardly of the pump housing thereby increasing and decreasing the volume of the further pump chamber, and wherein the plungers (3, 4) are configured for simultaneously being driven in opposite directions.

## Patentansprüche

1. Infusionspumpe (1), umfassend:
ein Pumpengehäuse (2);
einen Kolben (3), wobei das Pumpengehäuse und der Kolben eine Pumpenkammer (5) begrenzen, wobei der Kolben innerhalb des Pumpengehäuses nach innen und nach außen hin und her bewegbar ist, wodurch das Volumen der Pumpenkammer vergrößert und verkleinert wird;
einen Kolbenantrieb (7) zum Antreiben einer Verschiebung des Kolbens relativ zu dem Pumpengehäuse, wobei der Kolbenantrieb mit dem Kolben gekoppelt ist;
eine Zuführleitung (12-14), die mit der Pumpenkammer in Verbindung steht, um Infusionsflüssigkeit aus einem Behälter, wie beispielsweise einem IV-Beutel, der Pumpenkammer zuzuführen;
eine Abgabeleitung (15), die mit der Pumpenkammer in Verbindung steht, um Infusionsflüssigkeit aus der Pumpenkammer aufzunehmen und abzugeben;
mindestens ein Pumpventil (16), über das die Zuführleitung mit der Pumpenkammer in Verbindung steht, während die Abgabeleitung von der Pumpenkammer abgesperrt ist, wenn sich das Pumpventil in einem Infusionsaufnahmezustand befindet, und über das die Abgabeleitung mit der Pumpenkammer in Verbindung steht, während die Zuführleitung von der Pumpenkammer abgesperrt ist, wenn sich das Pumpventil in einem Infusionsabgabezustand befindet;
mindestens einen Pumpventilstellantrieb (17); und
eine Steuereinheit (21), die angeordnet und verbunden ist, um den Kolbenantrieb (7) und den Pumpenventilstellantrieb (17) zu steuern;
wobei der Kolbenantrieb (7), der Pumpenventilstellantrieb (17) und die Steuereinheit (21) Teil einer Basiseinheit (22) sind;
wobei das Pumpengehäuse (2), der Kolben (3), mindestens ein stromabwärtiger Abschnitt der Zuführleitung und mindestens ein stromaufwärtiger Abschnitt der Abgabeleitung und das mindestens eine Pumpenventil Teil einer Einwegeinheit sind, die lösbar an der Basiseinheit befestigt ist, die Kopplung des mindestens einen Pumpenventils mit dem mindestens einen Pumpenventilstellantrieb und die Kopplung des mindestens einen Kolbens mit dem Kolbenantrieb lösbar sind,
**gekennzeichnet durch:**
einem weiteren Kolben (4), wobei das Pumpengehäuse (2) und der weitere Kolben eine weitere Pumpenkammer (6) begrenzen, wobei der weitere Kolben innerhalb des Pumpengehäuses nach innen und nach außen hin und her bewegbar ist, wodurch das Volumen der weiteren Pumpenkammer vergrößert und verkleinert wird, und wobei der Kolbenantrieb (7) zum gleichzeitigen Antreiben der Kolben (3, 4) in entgegengesetzte Richtungen angeordnet ist.

2. Pumpe (1) nach Anspruch 1, wobei die Kopplung des mindestens einen Pumpenventils (16) mit dem mindestens einen Pumpenventilstellantrieb (17) und die Kopplung des mindestens einen Kolbens (3, 4) mit dem Kolbenantrieb (7) in einer gemeinsamen Richtung lösbar sind.

3. Pumpe (1) nach Anspruch 1 oder 2, wobei die Zuführleitung (12-14) und die Abgabeleitung (15) distale Enden von der Pumpenkammer (5) aufweisen, die für den Anschluss von Infusionsübertragungsschläuchen angeordnet sind.

4. Pumpe (1) nach einem der vorstehenden Ansprüche, wobei in der Einwegeinheit (23) die Zuführleitung (12-14) mindestens zwei stromaufwärtige Zuführleitungsabschnitte mit jeweils einem Anschluss für Infusionsübertragungsschläuche und einen stromabwärtigen gemeinsamen Zuführleitungsabschnitt umfasst, wobei ein Quellauswahlventil (38) bereitgestellt ist, um selektiv zu bewirken, dass ein erster oder ein zweiter der stromaufwärtigen Zuführleitungsabschnitte mit dem gemeinsamen Zuführleitungsabschnitt in Verbindung steht, und wobei die Basiseinheit (22) ferner einen Quellauswahlventil-Stellantrieb umfasst, der eine lösbar mit dem Quellauswahlventil gekoppelte Kupplung umfasst.

5. Pumpe (1) nach einem der vorstehenden Ansprüche, wobei die Einwegeinheit (23) ferner ein Entlüftungsventil in der Zuführleitung, eine von der Zuführleitung an dem Entlüftungsventil abzweigende Entlüftungsleitung, einen Blasendetektor und einen Blasendetektorsignalausgang umfasst, und wobei die Basiseinheit ferner einen Blasendetektorsignaleingang umfasst, der mit dem Blasendetektorsignalausgang in Verbindung steht, und einen Entlüftungsventil-Stellantrieb, der lösbar mit dem Entlüftungsventil gekoppelt ist, wobei die Steuereinheit angeschlossen und angeordnet ist, um das Entlüftungsventil in Reaktion auf ein Signal von dem Blasendetektor, das die Erkennung des Vorhandenseins von Luft darstellt, zu betätigen, sodass erkannte Luft über die Entlüftungsleitung abgegeben wird.

6. Pumpe (1) nach einem der vorstehenden Ansprüche, wobei mindestens eines der mindestens einen Pumpenventile ein Drehventil mit einem in dem Pumpengehäuse drehbaren Stopfen ist, wobei der Stopfen Durchgänge aufweist, die zum Bereitstellen einer Verbindung zwischen einer Einlassöffnung und einer Auslassöffnung, die dem Stopfen in mindestens einer Drehposition des Stopfens zugewandt sind, angeordnet sind, wobei der Stopfen eine Drehachse in einer Richtung aufweist, in der die Einwegeinheit von der Basiseinheit lösbar ist, und wobei der Stopfen durch einen entsprechenden des mindestens einen Pumpenventil-Stellantriebs betätigt und lösbar mit diesem gekoppelt werden kann.

7. Pumpe (1) nach Anspruch 6, wobei einerseits der Stopfen und andererseits der entsprechende Pumpenventil-Stellantrieb, der mit dem Stopfen lösbar gekoppelt werden kann, komplementär geformte Kupplungsflächen aufweisen, die um die Drehachse des Stopfens derart nicht rotationssymmetrisch sind, dass nur eine relative Drehstellung vorliegt, in der der entsprechende Pumpenventil-Stellantrieb mit dem Stopfen gekoppelt werden kann.

8. Pumpe (1) nach einem der vorstehenden Ansprüche, wobei die Kolben (3, 4) nur für eine gleichzeitige entgegengesetzte Bewegung miteinander gekoppelt sind, sodass Flüssigkeit nur aus einer Pumpenkammer eines der Kolben gefördert wird, während Flüssigkeit in eine Pumpenkammer des anderen Kolbens zugeführt wird.

9. Pumpe (1) nach Anspruch 8, wobei die Kolben (3, 4) mit einem Doppelarm gekoppelt sind, der eine Drehachse aufweist, die sich zentral zwischen den Kolben erstreckt.

10. Pumpe (1) nach Anspruch 9, wobei der Doppelarm ein Abschnitt eines Zahnrades mit einer Zahnstange ist, die sich entlang mindestens einer Kreissektion konzentrisch zu der Drehachse des Doppelarms erstreckt, wobei der Kolbenantrieb ferner ein Ritzel umfasst, das antriebsmäßig mit der Zahnstange in Eingriff steht.

11. Pumpe (1) nach Anspruch 9 oder 10, wobei das Pumpengehäuse (2) zwei Pumpengehäuseteile einschließt, die jeweils eine der Pumpenkammern (5, 6) begrenzen, wobei die Pumpengehäuseteile in der Nähe der Enden, die distal von den mit den Kolben (3, 4) in Eingriff stehenden Kupplungen angeordnet sind, schwenkbar aufgehängt sind.

12. Pumpe (1) nach einem der vorstehenden Ansprüche, wobei das Pumpenventil ein Vierwegeventil ist, in dem Anschlüsse angeordnet sind, um abwechselnd zu bewirken, dass eine der Pumpenkammern (5, 6) mit der Zuführleitung in Verbindung steht und zu bewirken, dass die andere der Pumpenkammern mit der Abgabeleitung in Verbindung steht und umgekehrt.

13. Einwegeinheit (23) zur Verwendung in einer Infusionspumpe (1), wobei die Einwegeinheit Folgendes umfasst:
ein Pumpengehäuse (2);
einen Kolben (3), wobei das Pumpengehäuse und der Kolben eine Pumpenkammer (5) begrenzen, wobei der Kolben innerhalb des Pumpengehäuses nach innen und nach außen hin und her bewegbar ist, wodurch das Volumen der Pumpenkammer vergrößert und verkleinert wird;
eine Zuführleitung (12-14), die mit der Pumpenkammer in Verbindung steht, um Infusionsflüssigkeit aus einem Behälter, wie beispielsweise einem IV-Beutel, der Pumpenkammer zuzuführen;
eine Abgabeleitung (15), die mit der Pumpenkammer in Verbindung steht, um Infusionsflüssigkeit aus der Pumpenkammer aufzunehmen und abzugeben; und
mindestens ein Pumpventil (16), über das die Zuführleitung mit der Pumpenkammer in Verbindung steht, während die Abgabeleitung von der Pumpenkammer abgesperrt ist, wenn sich das Pumpventil in einem Infusionsaufnahmezustand befindet, und über das die Abgabeleitung mit der Pumpenkammer in Verbindung steht, während die Zuführleitung von der Pumpenkammer abgesperrt ist, wenn sich das Pumpventil in einem Infusionsabgabezustand befindet;
wobei das mindestens eine Pumpenventil (16) zum lösbaren Koppeln mit mindestens einem Pumpenventilstellantrieb (17) und der mindestens eine Kolben zum lösbaren Koppeln mit einem Kolbenantrieb (7) angeordnet ist,
**gekennzeichnet durch:**
einem weiteren Kolben (4), wobei das Pumpengehäuse (2) und der weitere Kolben (4) eine weitere Pumpenkammer (6) begrenzen, wobei der weitere Kolben innerhalb des Pumpengehäuses nach innen und nach außen hin und her bewegbar ist, wodurch das Volumen der weiteren Pumpenkammer vergrößert und verkleinert wird, und wobei die Kolben (3, 4) dazu konfiguriert sind, gleichzeitig in entgegengesetzte Richtungen angetrieben zu werden.

## Revendications

1. Pompe à perfusion (1) comprenant :
un boîtier de pompe (2) ;
un piston (3), dans laquelle le boîtier de pompe et le piston sont reliés à une chambre de pompe (5), le piston est mobile selon un mouvement de va-et-vient vers l'intérieur et vers l'extérieur du boîtier de pompe, augmentant et diminuant ainsi le volume de la chambre de pompe ;
un entraînement de piston (7) pour entraîner le déplacement du piston par rapport au boîtier de pompe, l'entraînement de piston étant couplé au piston ;
un conduit d'alimentation (12-14) communiquant avec la chambre de pompe pour distribuer le liquide de perfusion à partir d'un réservoir, tel qu'un sachet pour perfusion intraveineuse, dans la chambre de pompe ;
un conduit de distribution (15) communiquant avec la chambre de pompe pour recevoir et distribuer le liquide de perfusion à partir de la chambre de pompe ;
au moins une valve de pompe (16) par laquelle le conduit d'alimentation communique avec la chambre de pompe, alors que le conduit de distribution est coupé de la chambre de pompe, si la valve de pompe est dans une condition d'admission de perfusion et par laquelle le conduit de distribution communique avec la chambre de pompe, alors que le conduit d'alimentation est coupé de la chambre de pompe, si la valve de pompe est dans une condition de distribution de perfusion ;
au moins un actionneur de valve de pompe (17) ; et
une unité de commande (21) agencée et raccordée pour commander l'entraînement de piston (7) et l'actionneur de valve de pompe (17) ;
dans lequel l'entraînement de piston (7), l'actionneur de valve de pompe (17) et l'unité de commande (21) font partie d'une unité de base (22) ;
dans lequel le boîtier de pompe (2), le piston (3), au moins une partie en aval du conduit d'alimentation et au moins une partie en amont du conduit de distribution et la au moins une valve de pompe font partie d'une unité jetable fixée, de manière amovible, à l'unité de base, le couplage de la au moins une valve de pompe au au moins un actionneur de valve de pompe et le couplage du au moins un piston à l'entraînement de piston étant amovibles,
**caractérisée par** :
un autre piston (4), dans laquelle le boîtier de pompe (2) et l'autre piston sont reliés à une autre chambre de pompe (6), l'autre piston est mobile selon un mouvement de va-et-vient vers l'intérieur et vers l'extérieur du boîtier de pompe augmentant et réduisant ainsi le volume de l'autre chambre de pompe, et dans laquelle l'entraînement de piston (7) est agencé pour entraîner simultanément les pistons (3, 4) dans des directions opposées.

2. Pompe (1) selon la revendication 1, dans laquelle le couplage de la au moins une valve de pompe (16) au au moins un actionneur de valve de pompe (17) et le couplage du au moins un piston (3, 4) à l'entraînement de piston (7) sont amovibles dans une direction commune.

3. Pompe (1) selon la revendication 1 ou 2, dans laquelle le conduit d'alimentation (12-14) et le conduit de distribution (15) ont des extrémités distales par rapport à la chambre de pompe (5) qui sont agencées pour le raccordement d'un tube de transfert de perfusion.

4. Pompe (1) selon l'une quelconque des revendications précédentes, dans laquelle, dans l'unité jetable (23), le conduit d'alimentation (12-14) comprend au moins deux sections de conduit d'alimentation en amont, chacune avec un raccordement pour le tube de transfert de perfusion, et une section de conduit d'alimentation commune en aval, une valve de sélection de source (38) étant prévue pour amener sélectivement une première ou une deuxième section de conduit d'alimentation en amont à communiquer avec la section de conduit d'alimentation commune, et dans laquelle l'unité de base (22) comprend en outre un actionneur de valve de sélection de source comprenant un couplage couplé, de manière amovible, à la valve de sélection de source.

5. Pompe (1) selon l'une quelconque des revendications précédentes, dans laquelle l'unité jetable (23) comprend en outre une valve de purge dans le conduit de distribution, un conduit de purge se séparant du conduit de distribution au niveau de la valve de purge, un détecteur de bulle et un orifice de sortie de signal de détecteur de bulle, et dans laquelle l'unité de base comprend en outre une entrée de signal de détecteur de bulle en communication avec l'orifice de sortie de signal de détecteur de bulle et un actionneur de valve de purge couplé, de manière amovible, à la valve de purge, dans laquelle l'unité de commande est raccordée et agencée pour actionner la valve de purge en réponse à un signal provenant de détecteur de bulle représentant la détection de présence d'air de sorte que l'air détecté est déchargé via le conduit de purge.

6. Pompe (1) selon l'une quelconque des revendications précédentes, dans laquelle au moins une de ladite au moins une valve de pompe est une valve rotative avec un bouchon rotatif dans le boîtier de pompe, le bouchon ayant des passages agencés pour fournir la communication entre un orifice d'entrée et un orifice de sortie des orifices faisant face au bouchon dans au moins une position de rotation du bouchon, le bouchon ayant un axe de rotation dans une direction dans laquelle l'unité jetable est amovible de l'unité de base, et dans laquelle le bouchon peut être actionné par et être couplé, de manière amovible, à un actionneur correspondant dudit au moins un actionneur de valve de pompe.

7. Pompe (1) selon la revendication 6, dans laquelle le bouchon, d'une part, et l'actionneur de valve de pompe correspondant qui peut être couplé, de manière amovible, au bouchon, d'autre part, ont des faces de couplage de forme complémentaire, qui sont non symétriques en rotation autour de l'axe de rotation du bouchon, de sorte qu'il n'y a qu'une position de rotation relative dans laquelle l'actionneur de valve de pompe correspondant peut être couplé au bouchon.

8. Pompe (1) selon l'une quelconque des revendications précédentes, dans laquelle les pistons (3, 4) sont couplés entre eux uniquement pour le mouvement opposé simultané, de sorte que le liquide est distribué à partir d'une chambre de pompe de l'un des pistons uniquement, alors que le liquide est amené à une chambre de pompe de l'autre des pistons.

9. Pompe (1) selon la revendication 8, dans laquelle les pistons (3, 4) sont couplés à un double bras ayant un axe de rotation s'étendant de manière centrale entre les pistons.

10. Pompe (1) selon la revendication 9, dans laquelle le double bras est une partie d'une roue dentée ayant une crémaillère dentée s'étendant le long d'au moins une section d'un cercle concentrique avec l'axe de rotation du double bras, l'entraînement de piston comprenant en outre un pignon mettant en prise, par entraînement, la crémaillère dentée.

11. Pompe (1) selon la revendication 9 ou 10, dans laquelle le boîtier de pompe (2) comprend deux parties de boîtier de pompe reliant chacune l'une des chambres de pompe (5, 6), dans laquelle les parties de boîtier de pompe sont suspendues, de manière pivotante, à côté des extrémités distales par rapport aux couplages mettant en prise les pistons (3, 4).

12. Pompe (1) selon l'une quelconque des revendications précédentes, dans laquelle la valve de pompe est une valve à quatre voies dans laquelle des orifices sont agencés pour amener, de manière alternée, l'une des chambres de pompe (5, 6) à communiquer avec le conduit de distribution et amener l'autre des chambres de pompe à communiquer avec le conduit d'alimentation et vice versa.

13. Unité jetable (23) destinée à être utilisée dans une pompe à perfusion (1), l'unité jetable comprenant :
un boîtier de pompe (2) ;
un piston (3), dans laquelle le boîtier de pompe et le piston sont reliés à une chambre de pompe (5), le piston est mobile, selon un mouvement de va-et-vient vers l'intérieur et vers l'extérieur du boîtier de pompe, augmentant et réduisant ainsi le volume de la chambre de pompe ;
un conduit d'alimentation (12-14) communiquant avec la chambre de pompe pour distribuer le liquide de perfusion à partir d'un réservoir, tel qu'un sachet pour perfusion intraveineuse, dans la chambre de pompe ;
un conduit de distribution (15) communiquant avec la chambre de pompe pour recevoir et distribuer le liquide de perfusion à partir de la chambre de pompe ; et
au moins une valve de pompe (16) par laquelle le conduit d'alimentation communique avec la chambre de pompe, alors que le conduit de distribution est coupé de la chambre de pompe, si la valve de pompe est dans une condition d'admission de perfusion et par laquelle le conduit de distribution communique avec la chambre de pompe, alors que le conduit d'alimentation est coupé de la chambre de pompe, si la valve de pompe est dans une condition de distribution de perfusion ;
dans laquelle la au moins une valve de pompe (16) est agencée pour se coupler, de manière amovible, à au moins un actionneur de valve de pompe (17) et le au moins un piston est agencé pour le couplage amovible à un entraînement de piston (7),
**caractérisée par** :
un autre piston (4), dans laquelle le boîtier de pompe (2) et l'autre piston (4) sont reliés à une autre chambre de pompe (6), l'autre piston est mobile selon un mouvement de va-et-vient vers l'intérieur et vers l'extérieur du boîtier de pompe augmentant et réduisant ainsi le volume de l'autre chambre de pompe, et dans laquelle les pistons (3, 4) sont configurés pour être entraînés simultanément dans des directions opposées.
